# EUROPEAN PATENT APPLICATION

(11) **EP 4 389 770 A1**
(43) Date of publication of application: **26.06.2024**
(21) Application number: 22857848.0
(22) Date of filing: 17.08.2022
(51) Int. Cl.: C07K 16/46, C12N 15/13, A61K 39/395, A61P 35/00, G01N 33/68

(54) **BISPECIFIC ANTIBODY AND USE THEREOF**

(30) Priority: 18.08.2021 CN 202110947802
(71) Applicant: Biotheus Inc., Tangjiawan Town, Xiangzhou District Zhuhai, Guangdong 519080 (CN)
(72) Inventor: LUO, Yi, Zhuhai, Guangdong 519080 (CN); CHEN, Junying, Zhuhai, Guangdong 519080 (CN); MIAO, Xiaoniu, Zhuhai, Guangdong 519080 (CN); HUANG, Weifeng, Zhuhai, Guangdong 519080 (CN); YUAN, Zhijun, Zhuhai, Guangdong 519080 (CN); PENG, Shaogang, Zhuhai, Guangdong 519080 (CN); TSUN, Andy, Zhuhai, Guangdong 519080 (CN)
(74) Representative: Cabinet Nony
(86) International application number: PCT/CN2022/113031
(87) International publication number: WO 2023/020537

(57) **Abstract**

The present application relates to a bispecific antibody and a use thereof, and also to an immunoconjugate and a pharmaceutical composition comprising the bispecific antibody. The present application further relates to uses of the bispecific antibody and immunoconjugate and pharmaceutical composition comprising the bispecific antibody. The bispecific antibody prepared in the present application can recognize or bind a cell expressing Claudin 18.2 and/or recognize or bind a cell expressing 4-1BB. Moreover, tumors can be prevented and/or treated in a subject.

## Description

### Technical Field

The present application belongs to the field of biomedical technology. More specifically, the present application relates to a bispecific antibody, as well as immunoconjugate and pharmaceutical composition containing the bispecific antibody. The present application also relates to a use of the bispecific antibody, as well as immunoconjugate and pharmaceutical composition containing the bispecific antibody.

### Background

Gastric cancer is one of the most prevalent cancers worldwide, with over 1 million new cases diagnosed in 2018. China bears a high incidence of gastric cancer, ranking second in malignant tumor occurrences according to recent data from the National Cancer Registration Center. The overall prognosis of gastric cancer is poor, marked by a 5-year survival rate ranging from 10% to 30%, and the 5-year survival rate of patients with advanced gastric cancer is less than 10%. In normal tissues, Claudin-18 isoform 2 (Claudin 18.2) exclusively expresses in gastric tissue cells, serving as a membrane-bound protein contributing to the formation of zonules occludens. However, Claudin 18.2 is up-regulated in various tumor cells, including gastric cancer and pancreatic cancer. Zolbetuximab (IMAB362), a monoclonal antibody targeting Claudin 18.2, has shown efficacy and minor toxicity in clinical studies.

Lymphocytes express a variety of costimulatory receptors, such as CD27, 4-1BB (CD137), OX40 (CD134), and GITR (CD357) in the tumor necrosis factor receptor superfamily. When activated, these receptors enhance immune effects and memory responses. Targeting these receptors with agonist antibodies effectively activates lymphocytes, enhancing their anti-tumor effects. 4-1BB, a crucial co-stimulatory receptor for T cells and NK cells, shows promising anticancer effects when combined with immune checkpoint inhibitors, particularly in tumors with poorer immunogenicity. However, severe target-related liver toxicity was observed during the clinical development of 4-1BB monoclonal antibody agonists (e.g., Urelumab), proving evidence of safety risks associated with systemic activation of immune cells through the 4-1BB receptor.

Therefore, there arises a need for a bispecific antibody capable of effectively activating immune cells against tumors (e.g., tumors expressing Claudin 18.2) while eliminating possible liver-related toxicity.

### Contents of the present invention

The inventors of the present application prepared a bispecific antibody through extensive experiments and repeated exploration. Mediated by Claudin 18.2 expressed by tumor cells, it specifically activates the 4-1BB receptor signaling pathway of immune cells, thereby effectively activating the anti-tumor effect of immune cells and eliminating possible systemic activation toxicity.

Therefore, in a first aspect, the present application provides a bispecific antibody, which comprises a Claudin 18.2 binding entity and a 4-1 BB binding entity, wherein the Claudin 18.2 binding entity comprises two pairs of identical immunoglobulin chains, wherein, each pair of immunoglobulin chains has a light chain and a heavy chain, the heavy chain comprises a heavy chain variable region and a heavy chain constant region; the light chain comprises a light chain variable region and a light chain constant region; the 4-1 BB binding entity comprises a heavy chain variable region; and, the heavy chain variable region of the 4-1 BB binding entity is connected to the C-terminus of the heavy chain of the Claudin 18.2 binding entity; wherein,
the Claudin 18.2 binding entity comprises: a VH CDR1 or variant thereof, a VH CDR2 or variant thereof, and a VH CDR3 or variant thereof contained in the heavy chain variable region (VH) as set forth in SEQ ID NO: 1; and/or, a VL CDR1 or variant thereof, a VL CDR2 or variant thereof, and a VL CDR3 or variant thereof contained in the light chain variable region (VL) as set forth in SEQ ID NO: 2;
the 4-1 BB binding entity comprises: a VH CDR1 or variant thereof, a VH CDR2 or variant thereof, and a VH CDR3 or variant thereof contained in the heavy chain variable region (VH) as set forth in SEQ ID NO: 3 or 25;
wherein, the variant has a substitution, deletion or addition of one or several amino acids (e.g., a substitution, deletion or addition, such as a conservative substitution, of 1, 2 or 3 amino acids) as compared to the sequence from which it is derived.

In certain embodiments, the substitution is a conservative substitution.

In certain embodiments, the three CDRs contained in the VH and/or the three CDRs contained in the VL are defined by the Kabat, IMGT or Chothia numbering system.

In certain embodiments, the antibody or antigen-binding fragment thereof as described above has one or two characteristics selected from the following:
(1) the Claudin 18.2 binding entity comprises:
   a heavy chain variable region (VH) comprising the following 3 complementarity determining regions (CDRs): a VH CDR1 with the sequence as set forth in SEQ ID NO: 9, a VH CDR2 with the sequence as set forth in SEQ ID NO: 10, and a VH CDR3 with the sequence as set forth in SEQ ID NO: 11; and/or a light chain variable region (VL) comprising the following 3 complementarity determining regions (CDRs): a VL CDR1 with the sequence as set forth in SEQ ID NO: 12, a VL CDR2 with the sequence as set forth in SEQ ID NO: 13, and a VL CDR3 with the sequence as set forth in SEQ ID NO: 14; wherein the CDRs are defined by the IMGT numbering system; or
(2) the 4-1 BB binding entity comprises:
   a heavy chain variable region (VH) comprising the following 3 complementarity determining regions (CDRs): a VH CDR1 with the sequence as set forth in SEQ ID NO: 15, a VH CDR2 with the sequence as set forth in SEQ ID NO: 16, and a VH CDR3 with the sequence as set forth in SEQ ID NO: 17; wherein the CDRs are defined by the IMGT numbering system.

In another aspect, the present application provides a bispecific antibody, which comprises a Claudin 18.2 binding entity and a 4-1 BB binding entity, wherein the Claudin 18.2 binding entity comprises two pairs of identical immunoglobulin chains, wherein, each pair of immunoglobulin chains has a light chain and a heavy chain, the heavy chain comprises a heavy chain variable region and a heavy chain constant region; the light chain comprises a light chain variable region and a light chain constant region; the 4-1 BB binding entity comprises a heavy chain variable region; and, the heavy chain variable region of the 4-1 BB binding entity is connected to the C terminus of the heavy chain of the Claudin 18.2 binding entity;
wherein, the heavy chain variable region (VH) of the Claudin 18.2 binding entity comprises the following 3 complementarity determining regions (CDRs):
   (i) a VH CDR1, which consists of the following sequence: SEQ ID NO: 9, or a sequence having a substitution, deletion or addition of one or several amino acids (e.g., a substitution, deletion or addition of 1, 2 or 3 amino acids) as compared thereto,
   (ii) a VH CDR2, which consists of the following sequence: SEQ ID NO: 10, or a sequence having a substitution, deletion or addition of one or several amino acids (e.g., a substitution, deletion or addition of 1, 2 or 3 amino acids) as compared thereto, and
   (iii) a VH CDR3, which consists of the following sequence: SEQ ID NO: 11, or a sequence having a substitution, deletion or addition of one or several amino acids (e.g., a substitution, deletion or addition of 1, 2 or 3 amino acids) as compared thereto,
the light chain variable region (VL) of the Claudin 18.2 binding entity comprises the following 3 complementarity determining regions (CDRs):
   (iv) a VL CDR1, which consists of the following sequence: SEQ ID NO: 12, or a sequence having a substitution, deletion or addition of one or several amino acids (e.g., a substitution, deletion or addition of 1, 2 or 3 amino acids) as compared thereto,
   (v) a VL CDR2, which consists of the following sequence: SEQ ID NO: 13, or a sequence having a substitution, deletion or addition of one or several amino acids (e.g., a substitution, deletion or addition of 1, 2 or 3 amino acids) as compared thereto, and
   (vi) a VL CDR3, which consists of the following sequence: SEQ ID NO: 14, or a sequence having a substitution, deletion or addition of one or several amino acids (e.g., a substitution, deletion or addition of 1, 2 or 3 amino acids) as compared thereto;
the heavy chain variable region of the 4-1 BB binding entity comprises the following 3 complementarity determining regions (CDRs):
   (vii) a VH CDR1, which consists of the following sequence: SEQ ID NO: 15, or a sequence having a substitution, deletion or addition of one or several amino acids (e.g., a substitution, deletion or addition of 1, 2 or 3 amino acids) as compared thereto,
   (viii) a VH CDR2, which consists of the following sequence: SEQ ID NO: 16, or a sequence having a substitution, deletion or addition of one or several amino acids (e.g., a substitution, deletion or addition of 1, 2 or 3 amino acids) as compared thereto, and
   (ix) a VH CDR3, which consists of the following sequence: SEQ ID NO: 17, or a sequence having a substitution, deletion or addition of one or several amino acids (e.g., a substitution, deletion or addition of 1, 2 or 3 amino acids) as compared thereto.

In certain embodiments, the CDRs as described in any of (i) to (ix) are defined according to the Kabat, IMGT, or Chothia numbering systems.

In certain embodiments, the Claudin 18.2 binding entity comprises: the following 3 heavy chain CDRs: a VH CDR1 with the sequence as set forth in SEQ ID NO: 9, a VH CDR2 with the sequence as set forth in SEQ ID NO: 10, a VH CDR3 with the sequence as set forth in SEQ ID NO: 11; and/or, the following 3 light chain CDRs: a VL CDR1 with the sequence as set forth in SEQ ID NO: 12, a VL CDR2 with the sequence as set forth in SEQ ID NO: 13, and a VL CDR3 with the sequence as set forth in SEQ ID NO: 14.

In certain embodiments, the heavy chain variable region (VH) of the 4-1 BB binding entity comprises: the following 3 heavy chain CDRs: a VH CDR1 with the sequence as set forth in SEQ ID NO: 15, a VH CDR2 with the sequence as set forth in SEQ ID NO: 16, and a VH CDR3 with the sequence as set forth in SEQ ID NO: 17.

In certain embodiments, the heavy chain variable region further comprises a framework region sequence derived from human (e.g., human immunoglobulin).

In certain embodiments, the Claudin 18.2 binding entity comprises:
(a) a heavy chain variable region (VH) comprising an amino acid sequence selected from the following:
   (i) a sequence as set forth in SEQ ID NO: 1;
   (ii) a sequence having a substitution, deletion or addition of one or several amino acids (e.g., a substitution, deletion or addition of 1, 2, 3, 4 or 5 amino acids) as compared to the sequence as set forth in SEQ ID NO: 1; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 1;
   and/or,
(b) a light chain variable region (VL) comprising an amino acid sequence selected from the following:
   (iv) a sequence as set forth in SEQ ID NO: 2;
   (v) a sequence having a substitution, deletion or addition of one or several amino acids (e.g., a substitution, deletion or addition of 1, 2, 3, 4 or 5 amino acids) as compared to the sequence as set forth in SEQ ID NO: 2; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 2.

In certain embodiments, the substitution described in (ii) or (v) is a conservative substitution.

In certain embodiments, the Claudin 18.2 binding entity comprises: a VH having the sequence as set forth in SEQ ID NO: 1 and a VL having the sequence as set forth in SEQ ID NO: 2.

In certain embodiments, the 4-1 BB binding entity comprises:
(a) a heavy chain variable region (VH) comprising an amino acid sequence selected from the following:
   (i) a sequence as set forth in SEQ ID NO: 3 or 25;
   (ii) a sequence having a substitution, deletion or addition of one or several amino acids (e.g., a substitution, deletion or addition of 1, 2, 3, 4 or 5 amino acids) as compared to the sequence as set forth in SEQ ID NO: 3 or 25; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 3 or 25.

In certain embodiments, the substitution described in (ii) is a conservative substitution.

The single domain antibody of the present application is not limited to specific biological sources or specific preparation methods. For example, the single domain antibody of the present application can be obtained: (1) by "humanizing" a naturally occurring VHH domain or by expressing a nucleic acid encoding such a humanized VHH domain; (2) using synthetic or semisynthetic techniques to prepare proteins, polypeptides or other amino acid sequences; (3) by using nucleic acid synthesis techniques to prepare a nucleic acid encoding the single domain antibody, and then expressing the nucleic acid thus obtained; and/or (4) by any combination of the foregoing.

In certain embodiments, the Claudin 18.2 binding entity further comprises:
(a) a human immunoglobulin heavy chain constant region (CH) or variant thereof, wherein the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived; and
(b) a human immunoglobulin light chain constant region (CL) or variant thereof, wherein the variant has a conservative substitution of up to 20 amino acids (e.g., a conservative substitution of up to 15, up to 10, or up to 5 amino acids; for example, a conservative substitution of 1, 2, 3, 4 or 5 amino acids) as compared to the wild-type sequence from which it is derived.

In certain embodiments, the heavy chain constant region is an IgG heavy chain constant region, such as an IgGl, IgG2, IgG3 or IgG4 heavy chain constant region.

In certain embodiments, the light chain constant region is a κ or λ light chain constant region.

In certain embodiments, the Claudin 18.2 binding entity comprises a light chain constant region (CL) as set forth in SEQ ID NO: 8.

In certain embodiments, the bispecific antibody further comprises an Fc fragment that does not bind to an Fc receptor (FcR); alternatively, comprises an Fc fragment that has reduced effector function, wherein the effector function is antibody-dependent cell-mediated cytotoxicity (ADCC), complement-dependent cytotoxicity (CDC), or antibody-dependent cellular phagocytosis (ADCP).

In certain embodiments, the Fc fragment is selected from any one of the following mutants:
(a) an IgG1 Fc fragment having L235E mutation;
(b) an IgG1 Fc fragment having L234A and/or L235A mutations;
(c) an IgG1 Fc fragment having P329G or P329A mutation;
(d) an IgG4 Fc fragment having F234A and/or L235A or L235E mutations;
(e) an IgG2 Fc fragment having H268Q, V309L, A330S and/or P331S mutations; or
(f) an IgG2 Fc fragments having V234A, G237A, P238S, H268A, V309L, A330S and/or P331S mutations.

In certain embodiments, the Fc fragment is an IgGl Fc fragment having L234A and L235A mutations.

In certain embodiments, the Fc fragment has the sequence as set forth in SEQ ID NO: 4.

In certain embodiments, the bispecific antibody further comprises a peptide linker, and the Claudin 18.2 binding entity and the 4-1 BB binding entity are connected by the peptide linker.

In certain embodiments, the C-terminus of the heavy chain constant region of the Claudin 18.2 binding entity is connected to the N-terminus of the heavy chain variable region of the 4-1 BB binding entity via a peptide linker.

The peptide linker can comprise any amino acid. In certain embodiments, the peptide linker comprises glycine (G) and serine (S). In certain embodiments, the peptide linker has a length of 1 to 50 amino acids. The peptide linker is long enough to provide a sufficient degree of flexibility to allow the protein to fold properly. The length and amino acid composition of the peptide linker can be easily selected by those skilled in the art.

In certain embodiments, the peptide linker has the sequence as set forth in SEQ ID NO: 5 or 26.

Various methods for preparing bispecific antibodies are disclosed in the prior art. For example, US5989830 discloses a method for preparing bispecific antibodies, in which bispecific antibody fragments are obtained by expressing bicistronic vectors encoding two polypeptide chains, and one polypeptide chains of bispecific antibody has two VHs connected in series through a peptide linker (i.e., VH1-peptide linker-VH2).

In certain embodiments, the heavy chain of the bispecific antibody has the amino acid sequence as set forth in SEQ ID NO: 6, 27, 28 or 29.

In certain embodiments, the light chain of the bispecific antibody has the amino acid sequence as set forth in SEQ ID NO: 7.

In a second aspect, the present application provides an isolated nucleic acid molecule, which encodes the bispecific antibody, or heavy chain and/or light chain thereof, or heavy chain variable region and/or the light chain variable region of Claudin 18.2 binding entity, or heavy chain variable region of 4-1 BB binding entity as described above. Such nucleic acid molecule is not limited by the method of its production and can be obtained using genetic engineering recombinant techniques or chemical synthesis methods.

In a third aspect, the present application provides a vector, which comprises the isolated nucleic acid molecule as described above. In certain embodiments, the vector is a cloning vector or an expression vector. In certain preferred embodiments, the vector of the present invention is, for example, a plasmid, a cosmid, a phage, or the like.

In a fourth aspect, the present application provides a host cell, which comprises the isolated nucleic acid molecule as described above or the vector as described above. Such host cell includes, but is not limited to, prokaryotic cell such as *E. coli* cell, eukaryotic cell such as yeast cell, insect cell, plant cell, and animal cell (e.g., mammalian cell, such as mouse cell, human cell, etc.). The cell of the present invention may also be a cell line, such as HEK293 cell.

In another aspect, the present application also provides a method for preparing the bispecific antibody of the present invention, which comprises culturing the host cell of the present invention under suitable conditions, and recovering the Claudin 18.2 binding entity and/or 4-1BB binding entity of the present invention from a cell culture.

Optionally, the Claudin 18.2 binding entity and the 4-1BB binding entity are linked via a peptide linker.

In another aspect, the present invention provides a kit, which comprises the bispecific antibody of the present invention. In certain preferred embodiments, the bispecific antibody of the present invention further comprises a detectable label. In certain preferred embodiments, the kit further comprises a second antibody that specifically recognizes the bispecific antibody of the present invention. In certain preferred embodiments, the second antibody further comprises a detectable label. Such detectable labels are well known to those skilled in the art and include, but are not limited to, radioactive isotopes, fluorescent substances, luminescent substances, colored substances, and enzymes (e.g., horseradish peroxidase) and the like.

In a fifth aspect, the present application provides an immunoconjugate, which comprises the bispecific antibody as described above and a therapeutic agent linked to the bispecific antibody.

In certain embodiments, the therapeutic agent is selected from cytotoxic agents.

In certain embodiments, the therapeutic agent is selected from the group consisting of alkylating agent, mitotic inhibitor, anti-tumor antibiotic, antimetabolite, topoisomerase inhibitor, tyrosine kinase inhibitor, radionuclide agent, and any combination thereof.

In certain embodiments, the immunoconjugate is an antibody-drug conjugate (ADC).

In a sixth aspect, the present application provides a pharmaceutical composition, which comprises the bispecific antibody as described above or the immunoconjugate as described above, and a pharmaceutically acceptable carrier and/or excipient.

In certain embodiments, the pharmaceutical composition further comprises an additional pharmaceutically active agent.

In certain embodiments, the additional pharmaceutically active agent is a drug with anti-tumor activity, such as an alkylating agent, a mitosis inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the bispecific antibody or immunoconjugate and the additional pharmaceutically active agent are provided as separate components or as components of the same composition.

In a seventh aspect, the present application provides a method for inhibiting the growth of a tumor cell expressing Claudin 18.2 and/or killing the tumor cell, which comprises contacting the tumor cell with an effective amount of the bispecific antibody as described above, or the immunoconjugate as described above, or the pharmaceutical composition as described above.

In an eighth aspect, the present application provides a use of the bispecific antibody as described above, or the immunoconjugate as described above, in the manufacture of a medicament for preventing and/or treating a tumor in a subject (e.g., a human).

In certain embodiments, the medicament further comprises an additional pharmaceutically active agent.

In certain embodiments, the additional pharmaceutically active agent is a drug with anti-tumor activity, such as an alkylating agent, a mitosis inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the tumor expresses Claudin 18.2.

In certain embodiments, the tumor involves a tumor cell expressing Claudin 18.2. In certain embodiments, the Claudin 18.2 is expressed on the surface of the tumor cell.

In certain embodiments, the tumor is selected from the group consisting of gastric cancer, liver cancer, biliary tract cancer, renal cell cancer, pancreatic cancer, non-small cell lung cancer, mesothelioma, ovarian cancer, testicular cancer, endometrial cancer, lung cancer, esophageal cancer, pancreatic cancer, bronchial cancer, breast cancer, ear-nose-throat (ENT) cancer, colon cancer, head-neck cancer, and gallbladder cancer.

In certain embodiments, the subject is a mammal, such as a human.

In the ninth aspect, the present application provides a use of the bispecific antibody as described above, or the immunoconjugate as described above, or the pharmaceutical composition as described above, in the manufacture of a kit, and the kit is used for:
(1) recognizing or binding to a cell expressing Claudin 18.2;
(2) recognizing or binding to a cell expressing 4-1BB;
(3) activating a NF-κB signaling pathway of a cell;
(4) inducing a cell (e.g., an immune cell) to activate tumor-killing activity;
(5) inducing a cell (e.g., an immune cell) to secrete a cytokine (e.g., IFNγ and IL-2);
(6) any combination of (1) to (5).

In certain embodiments, the immune cell is selected from the group consisting of B cell, T cell, and NK cell.

### Definition of Terms

In the present invention, unless otherwise stated, scientific and technical terms used herein have the meanings commonly understood by those skilled in the art. Moreover, the procedures used herein such as molecular genetics, nucleic acid chemistry, chemistry, molecular biology, biochemistry, cell culture, microbiology, cell biology, genomics and recombinant DNA are routine procedures widely used in the corresponding fields. Meanwhile, in order to better understand the present invention, definitions and explanations of relevant terms are provided below.

As used herein, the term "Claudin-18 (CLDN18)" refers to a transmembrane protein located on the cell membrane. The first exon of the human CLDN18 gene comprises two alleles, giving rise to two distinct splice variants: Claudin 18.1 and Claudin 18.2. Despite their closely related structures, Claudin 18.1 and Claudin 18.2 exhibit differential expression patterns in normal tissues and tumors. Claudin 18.2 is exclusively expressed in gastric mucosal tissue, and its expression is heightened in various cancers, including gastric, esophageal, and pancreatic cancer. This up-regulation of Claudin 18.2 is not limited to primary tumors; it is also prominently expressed in tumor metastases. Those skilled in the art can obtain the sequence of Claudin 18.2 through public databases (e.g., NCBI), for example, the sequence number NP_001002026.1 obtained from the NCBI database. In this article, "Claudin 18.2" may also be referred to as "CLND18.2".

As used herein, the term "4-1 BB" is also known as CD137, is a member of the tumor necrosis factor receptor superfamily 9 (TNFRSF9). It is primarily expressed in activated T cells, functioning as a T cell costimulatory molecule with its specific ligand being 4-1BBL. The interaction between 4-1 BB and 4-1BBL can stimulate T cell activation and proliferation. Those skilled in the art can obtain the sequence of 4-1BB through public databases (e.g., NCBI),.For example, refer to the sequence number NP_001552.2 in the NCBI database.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and define the antibody's isotype as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the interaction of immunoglobulin with host tissue or factor, including the binding of various cells of the immune system (e.g., effector cells) to the first component of classical complement system (C1q). The VH and VL regions can also be subdivided into regions of high variability called complementarity determining regions (CDRs), interspersed with more conservative regions called framework regions (FRs). Each VH and VL consists of 3 CDRs and 4 FRs arranged from the amino terminus to the carboxyl terminus in the following order: FR1, CDR1, FR2, CDR2, FR3, CDR3, FR4. The variable regions (VH and VL) of each heavy chain/light chain pair respectively form the antigen-binding site. The assignment of amino acids to each region or domain can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the terms "single domain antibody (sdAb)", "domain antibody" and "nanobody" are used interchangeably and refer to an antibody fragment composed of single variable domain (e.g. heavy chain variable region) in an antibody. Typically, single domain antibody, structural domain antibody or nanobody is composed of 4 framework regions and 3 complementarity determining regions, in which the 4 framework regions are FR1 to FR4, respectively, and the 3 complementarity determining regions are CDR1 to CDR3, respectively. In certain embodiments, the single domain antibody of the present application may have the structure of FR1-CDR1-FR2-CDR2-FR3-CDR3-FR4. These antibodies do not require a light chain variable region to bind antigen with high affinity and specificity. Compared with an antibody composed of heavy and light chains, a nanobody has high solubility and high stability to heat, pH, proteases and other deforming agents, and can be produced in large-scale with only single-chain expression.

As used herein, the term "bispecific antibody" refers to a conjugate formed by a first antibody (or fragment thereof) and a second antibody (or fragment thereof) or antibody analog through a coupling arm, and the conjugation method includes, but is not limited to, chemical reaction, gene fusion, and enzymatic method. The bispecific antibody can be linked or generated by various methods, see for example the methods of Songsivilai et al. (Clin. Exp. Immunol., 79:315-321 (1990)), and KosThe method of Telny et al. (J. Immunol., 148:1547-1553 (1992)).

As used herein, the term "humanized antibody" refers to a non-human antibody that has been genetically engineered, and its amino acid sequence has been modified to increase sequence homology to that of a human antibody. Generally speaking, all or part of the CDR regions of a humanized antibody come from a non-human antibody (donor antibody), and all or part of the non-CDR regions (e.g., FR) come from a human immunoglobulin (recipient antibody). Humanized antibodies usually retain the expected properties of the donor antibody, including but not limited to, antigen specificity, affinity, reactivity, ability to increase immune cell activity, ability to enhance immune response, etc. The donor antibody may be a camel-derived antibody with desired properties (e.g., antigen specificity, affinity, reactivity, ability to increase immune cell activity, and/or ability to enhance immune response).

As used herein, the term "binding entity" refers to any monomeric or multimeric protein or protein fragment that specifically binds to a target antigen. The term "binding entity" includes, but is not limited to, an antibody or binding portion thereof, such as an immunologically functional fragment.

As used herein, the term "peptide linker" refers to a peptide adapted to link to an antibody binding entity.

As used herein, the term "immunoglobulin" refers to a protein consisting of one or more polypeptides encoded by immunoglobulin genes. Immunoglobulins form the basic building blocks of antibodies. Antibodies are usually tetramers and are composed of two pairs of identical immunoglobulin chains, each pair having a light chain and a heavy chain. Within each pair of immunoglobulin chains, the light and heavy chain variable regions together are responsible for antigen binding, and the constant regions are responsible for antibody effector functions.

As used herein, the "light chains" of immunoglobulins may be classified into κ (kappa) and λ (lambda) light chains. Heavy chains can be classified as µ, δ, γ, α, or ε, and define the antibody's isotype as IgM, IgD, IgG, IgA, and IgE, respectively.

As used herein, the term "complementarity determining region" or "CDR" refers to those amino acid residues in a variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883) or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

As used herein, the term "framework region" or "FR" residues refers to those amino acid residues in an antibody variable region other than the CDR residues as defined above.

As used herein, the terms "monoclonal antibody", "monoclonal antibody" and "mAb" have the same meaning and are used interchangeably, and refer to an antibody a fragment of an antibody from a group of highly homologous antibody molecules, that is, a group of identical antibody molecules except for natural mutations that may occur spontaneously. Monoclonal antibodies are highly specific for a single epitope on an antigen. Polyclonal antibodies are relative to monoclonal antibodies, which usually contain at least two or more different kinds of antibodies, and these different antibodies usually recognize different epitopes on an antigen. Furthermore, the modifier "monoclonal" merely indicates that the antibody is characterized as being obtained from a highly homologous population of antibodies and is not construed as requiring any specific method to prepare the antibody.

The monoclonal antibody of the present invention can be prepared by a variety of techniques, such as hybridoma technology (see, for example, Kohler et al. Nature, 256:495, 1975), recombinant DNA technology (see, for example, U.S. Patent Application 4,816,567), or phage Antibody library technology (see, for example, Clackson et al. Nature 352: 624-628, 1991, or Marks et al. J. Mol. Biol. 222: 581-597, 1991).

As used herein, the term "subject" refers to a mammal, such as a primate mammal, such as a human. In certain embodiments, the subject (e.g., a human) has a tumor (e.g., a tumor expressing CLDN6 and/or CLDN9), or is at risk of suffering from a disease described above.

As used herein, the term "effective amount" refers to an amount sufficient to obtain, at least in part, a desired effect. For example, a prophylactically effective amount refers to an amount sufficient to prevent, arrest, or delay the occurrence of a disease (e.g., a tumor); a therapeutically effective amount refers to an amount sufficient to cure or at least partially prevent an existing disease and complications thereof in a patient. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner in which the drug is administered, and other treatments administered concurrently etc.

As used herein, the term "identity" is used to refer to the match of sequences between two polypeptides or between two nucleic acids. When a position in both sequences being compared is occupied by the same base or amino acid monomer subunit (e.g., a certain position in each of two DNA molecules is occupied by adenine, or a certain position in each of two polypeptides is occupied by lysine), then the molecules are identical at that position. "Percent identity" between two sequences is a function of the number of matching positions shared by the two sequences divided by the number of positions as compared × 100. For example, if 6 out of 10 positions of two sequences match, then the two sequences have an identify of 60%. For example, the DNA sequences CTGACT and CAGGTT have an identify of 50% (3 out of a total of 6 positions match). Typically, comparisons are made when two sequences are aligned to yield maximum identity. Such alignment can be accomplished using, for example, the method of Needleman et al. (1970) J. Mol. Biol. 48:443-453, which can be conveniently performed by a computer program such as the Align program (DNAstar, Inc.). In addition, the algorithm of E. Meyers and W. Miller (Comput. Appl Biosci., 4:11-17 (1988)), which has been integrated into the ALIGN program (version 2.0), can also be used to determine the percent identity between two amino acid sequences by using the PAM120 weight residue table as well as a gap length penalty of 12 and a gap penalty of 4. Furthermore, the algorithm of Needleman and Wunsch (J MoIBiol. 48:444-453 (1970)), which has been integrated into the GAP program of the GCG software package (available at www.gcg.com), can be used to the percent identity between two amino acid sequences by using the Blossum 62 matrix or the PAM250 matrix as well as a gap weight of 16, 14, 12, 10, 8, 6 or 4 and a length weight of 1, 2, 3, 4, 5 or 6.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions comprise those in which an amino acid residue is replaced with an amino acid residue having a similar side chain, for example, a residue that is physically or functionally similar to the corresponding amino acid residue (e.g., having similar size, shape, charge, chemical properties, including ability to form covalent bonds or hydrogen bonds, etc.) is used for substitution. Families of amino acid residues with similar side chains have been defined in the art. These families comprise amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine amino acids, proline, phenylalanine, methionine), β-branched side chains (e.g., threonine, valine, isoleucine) and aromatic side chains (e.g., tyrosine, Phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. NatlAcad. Set USA 94:412-417 (1997), which is incorporated herein by reference).

As used herein, the term "L234A mutation" refers to the mutation of amino acid at position 234 of the native Fc fragment from L to A. Similarly, "L235A mutation" refers to the mutation of amino acid at position 235 of the native Fc fragment from L to A.

### Beneficial effects of the present invention

Compared with the prior art, the bispecific antibody prepared in the present application can recognize or bind to cells expressing Claudin 18.2 and/or recognize or bind to cells expressing 4-1BB. Moreover, it can activate the NF-κB signaling pathway of cells, induce cells to activate tumor-killing activity, and can also induce cells to secrete cytokines (e.g., IFNγ and IL-2), and can prevent and/or treat a tumor in a subject. Moreover, the bispecific antibody has a more prominent effect in preventing and/or treating a tumor as compared with commercially available monoclonal antibodies or monoclonal antibodies used in combination.

In addition, when compared to monoclonal antibody targeting 4-1BB, the bispecific antibody in the present application reduces liver-related targeting effects. By using the tumor-associated antigen (TAA) Claudin 18.2 to cross-link the 4-1BB receptor that lacks Fc receptor (FcR) binding properties, the targeted anti-tumor efficacy without 4-1BB-related toxicity had been achieved. At the same time, the present application incorporated an Fc domain capable of effectively removing or reducing FcR binding and limiting Fc-mediated biological activity.

The embodiments of the present invention will be described in detail below with reference to the accompanying drawings and examples, but those skilled in the art will understand that the following drawings and examples are only used to illustrate the present invention and do not limit the scope of the present invention. Various objects and advantageous aspects of the present invention will become apparent to those skilled in the art from the accompanying drawings and the following detailed description of preferred embodiments.

### Brief Description of the Drawings

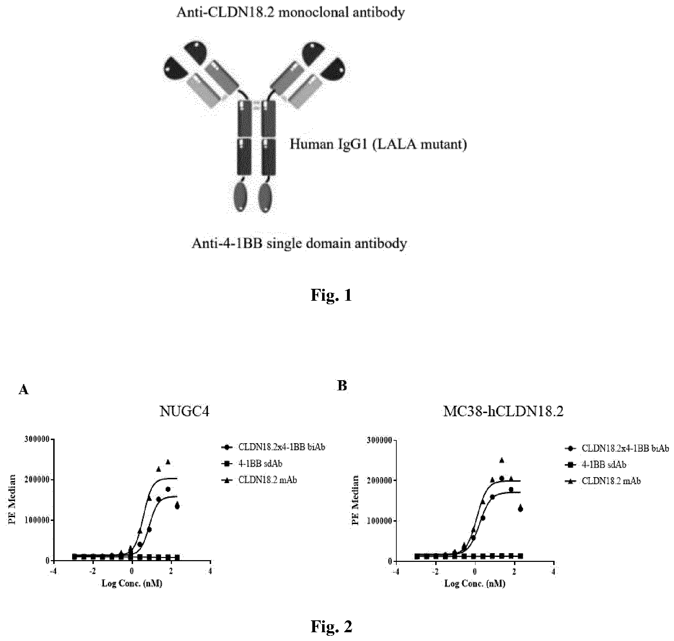

Fig. 1 shows a schematic diagram of the bispecific antibody targeting Claudin 18.2 and 4-1 BB in the present application.
Fig. 2 shows the results of the bispecific antibody of the present application binding to Claudin 18.2. Therein, Fig. 2A shows the results of the bispecific antibody in the present application binding to NUGC4 cells endogenously expressed Claudin 18.2; Fig. 2B shows the result of the bispecific antibody of the present application binding to human Claudin 18.2 overexpressed by MC38 cells; wherein, Claudin 18.2×4-1BB biAb was the bispecific antibody of the present application, 4-1BB sdAb was a 4-1BB single domain antibody, and Claudin 18.2 mAb was a Claudin 18.2 monoclonal antibody.

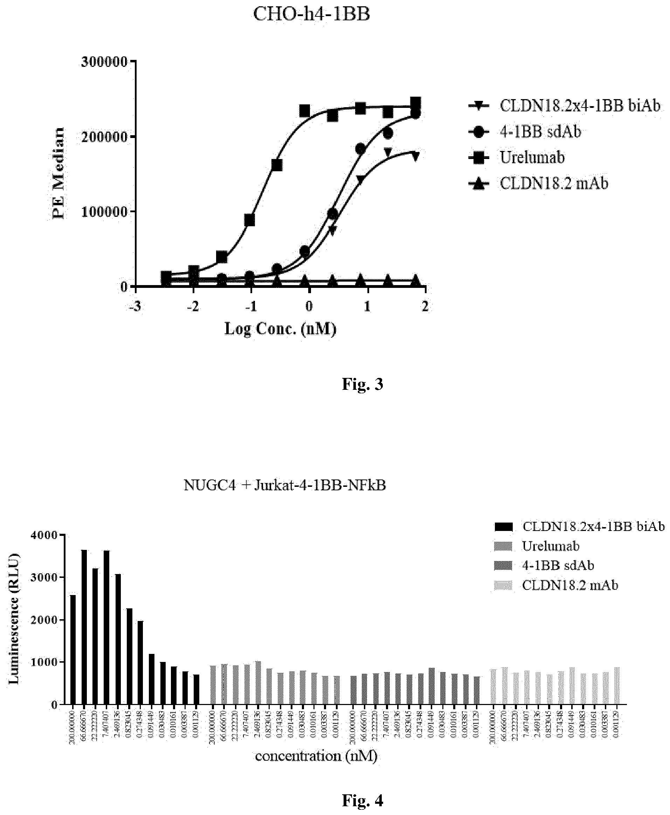

Fig. 3 shows the results of the bispecific antibodies of the present application binding to human 4-1BB overexpressed by CHO cells; wherein, Claudin 18.2×4-1BB biAb was the bispecific antibody of the present application, and 4-1BB sdAb was a 4-1BB single domain antibody, Claudin 18.2 mAb was a Claudin 18.2 monoclonal antibody, and Urelumab was a 4-1BB monoclonal antibody.
Fig. 4 shows the results of the bispecific antibody of the present application activating the NF-κB signaling pathway in Jurkat cells; wherein, Claudin 18.2×4-1BB biAb was the bispecific antibody of the present application, and 4-1BB sdAb was a 4-1BB single domain antibody, Claudin 18.2 mAb was a Claudin 18.2 monoclonal antibody, and Urelumab was a 4-1BB monoclonal antibody.

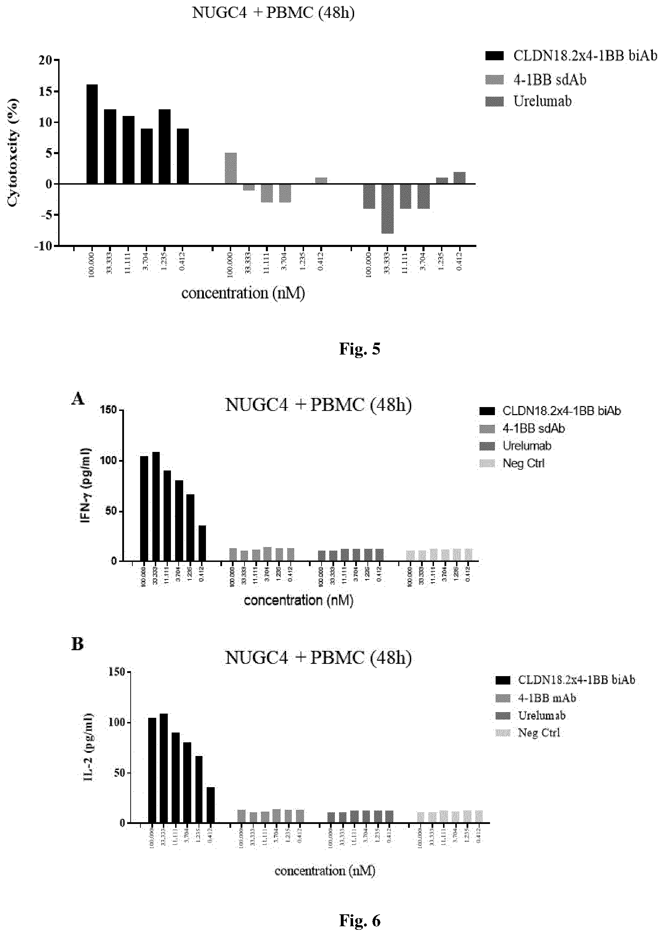

Fig. 5 shows the results of Claudin 18.2 positive tumor cells NUGC4 killing by peripheral blood mononuclear cells (PBMC) induced by the bispecific antibody of the present application; wherein, Claudin 18.2×4-1BB biAb was the bispecific antibody of the present application, 4-1BB sdAb was a 4-1BB single domain antibody, and Urelumab was a 4-1BB monoclonal antibody.
Fig. 6 shows the results of inducing the release of cytokines from PBMC by the bispecific antibody of the present application, wherein Fig. 6A shows the results of the release of cytokine IFNγ, and Fig. 6B shows the results of the release of cytokine IL-2; wherein, Claudin 18.2×4-1BB biAb was the bispecific antibody of the present application, 4-1BB sdAb was a 4-1BB single domain antibody, Urelumab was a 4-1BB monoclonal antibody, and Neg Ctrl was a negative control.

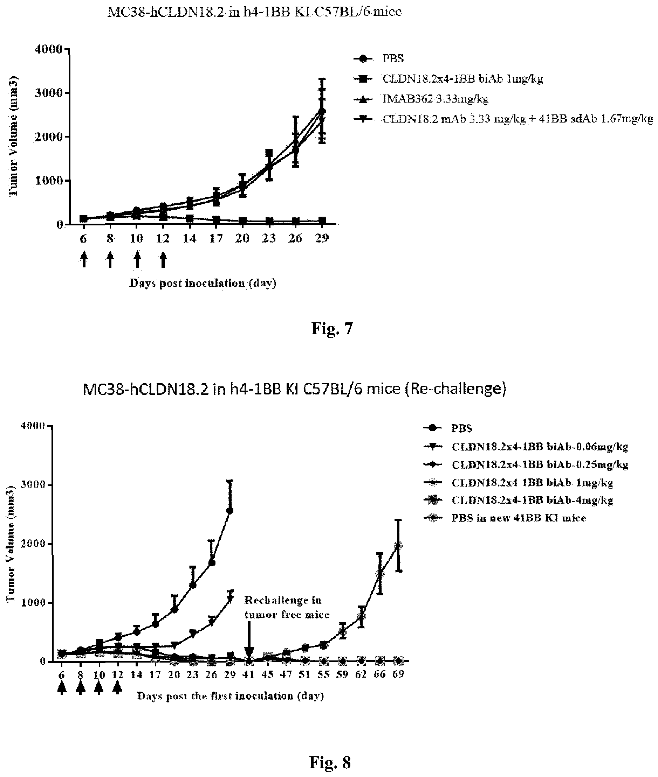

Fig. 7 shows the detection results of the tumor inhibitory activity of the bispecific antibody of the present application. Therein, PBS was a negative control, Claudin 18.2×4-1BB biAb was the bispecific antibody of the present application, IMAB362 was a Claudin 18.2 monoclonal antibody, and Claudin 18.2 mAb+4-1BB sdAb was a combination of two antibodies used to construct the bispecific antibody of the present application.
Fig. 8 shows the detection results of the tumor inhibitory memory effect of the bispecific antibody of the present application. Therein, PBS was a negative control, Claudin 18.2×4-1BB biAb was the bispecific antibody of the present application at different concentrations, and PBS in new 4-1BB KI mice was a negative control when tumor inoculation was performed again.

### Sequence Information

Information of partial sequences involved in the present invention is provided in Table 1 below.

**Table 1: Description of sequences**

| SEQ ID NO: | Description | Sequence |
|---|---|---|
| 1 | anti-Claudin 18.2 mAb heavy chain variable region | QVQLVQSGAEVKKPGASVKVSCKASGYTFTSYYMHWVRQAPGQ GLEWMGTIYPGNGDTSYNQKFQGRVTMTRDKSTSTVYMELSSLR SEDTAVYFCARGGYYGNSLDYWGQGTLVTVSS |
| 2 | anti- Claudin 18.2 mAb light chain variable region | DIVMTQSPDSLAVSLGERATINCKSSQSVLSSGNQKNYLAWYQQK PGQPPKLLIYWASTRESGVPDRFSGSGSGTDFTLTISSLQAEDVAV YYCQNAYYYPFTFGQGTKLEIK |
| 3 | 4-1BB heavy chain variable region of single domain antibody HZ-L-Yr-16-1-AB20ME | QVQLVESGGGVVQPGRSLRLSCAASGSTFSIVAMGWYRQAPGKQ RELVASIITGDGDTNYADSVKGRFTISRDNSKNTMYLQMNSLKPE DTAVYYCYARTGEGSSWLEGHEYDYWGQGTQVTVSS |
| 4 | Human IgG1 Fc amino acid sequence | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPEAAGGPSVFLFPPKPKDTLMIS RTPEVTCVVVDVSHEDPEVKFNWYVDGVEVHNAKTKPREEQYN STYRVVSVLTVLHQDWLNGKEYKCKVSNKALPAPIEKTISKAKG QPREPQVYTLPPSREEMTKNQVSLTCLVKGFYPSDIAVEWESNGQ PENNYKTTPPVLDSDGSFFLYSKLTVDKSRWQQGNVFSCSVMHE ALHNHYTQKSLSLSPGK |
| 5 | Flexible peptide sequence-1 | GGGGSGGGGSGGGGSGGGGS |
| 6 | Bispecific antibody Claudin 18.2x4-1BB biAb heavy chain | QVQLVQSGAEVKKPGASVKVSCKASGYTFTSYYMHWVRQAPGQ GLEWMGTIYPGNGDTSYNQKFQGRVTMTRDKSTSTVYMELSSLR SEDTAVYFCARGGYYGNSLDYWGQGTLVTVSSASTKGPSVFPLA PSSKSTSGGTAALGCLVKDYFPEPVTVSWNSGALTSGVHTFPAVL QSSGLYSLSSVVTVPSSSLGTQTYICNVNHKPSNTKVDKKVEPKSC DKTHTCPPCPAPEAAGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSR EEMTKNQVSLTCLVKGFYPSDIAVEWESNGQPENNYKTTPPVLDS DGSFFLYSKLTVDKSRWQQGNVFSCSVMHEALHNHYTQKSLSLS PGKGGGGSGGGGSGGGGSGGGGSQVQLVESGGGVVQPGRSLRLS CAASGSTFSIVAMGWYRQAPGKQRELVASIITGDGDTNYADSVK GRFTISRDNSKNTMYLQMNSLKPEDTAVYYCYARTGEGSSWLEG HEYDYWGQGTQVTVSS |
| 7 | Bispecific antibody Claudin 18.2x4-1BB biAb light chain | DIVMTQSPDSLAVSLGERATINCKSSQSVLSSGNQKNYLAWYQQK PGQPPKLLIYWASTRESGVPDRFSGSGSGTDFTLTISSLQAEDVAV YYCQNAYYYPFTFGQGTKLEIKRTVAAPSVFIFPPSDEQLKSGTAS VVCLLNNFYPREAKVQWKVDNALQSGNSQESVTEQDSKDSTYSL SSTLTLSKADYEKHKVYACEVTHQGLSSPVTKSFNRGEC |
| 8 | Human CL amino acid sequence | |
| 9 | anti-Claudin 18.2 mAb VH CDR1 | GYTFTSYY |
| 10 | anti-Claudin 18.2 mAb VH CDR2 | IYPGNGDT |
| 11 | anti-Claudin 18.2 mAb VH CDR3 | ARGGYYGNSLDY |
| 12 | anti-Claudin 18.2 mAb VL CDR1 | QSVLSSGNQKNY |
| 13 | anti-Claudin 18.2 mAb VL CDR2 | WAS |
| 14 | anti-Claudin 18.2 mAb VL CDR3 | QNAYYYPFT |
| 15 | HZ-L-Yr-16-1-AB20ME VH CDR1 | GSTFSIVA |
| 16 | HZ-L-Yr-16-1-AB20ME VH CDR2 | IITGDGDT |
| 17 | HZ-L-Yr-16-1-AB20ME VH CDR3 | YARTGEGSSWLEGHEYDY |
| 18 | Nucleotide sequence encoding bispecific body heavy chain | CAGGTGCAGCTGGTTCAGTCTGGCGCCGAAGTGAAGAAACCTG GCGCCTCTGTGAAGGTGTCCTGCAAGGCTTCCGGCTACACCTTT ACCAGCTACTACATGCACTGGGTCCGACAGGCCCCTGGACAAG GATTGGAGTGGATGGGCACCATCTATCCCGGCAACGGCGACAC CTCCTACAACCAGAAATTCCAGGGCAGAGTGACCATGACCAGA GACAAGTCCACCTCCACCGTGTACATGGAACTGTCCAGCCTGA GATCCGAGGATACCGCCGTGTACTTCTGTGCCAGAGGCGGCTA CTACGGCAACTCCCTGGATTATTGGGGCCAGGGCACACTGGTC ACCGTGTCCTCTGCTTCTACCAAGGGACCCAGCGTGTTCCCTCT GGCTCCTTCCAGCAAGTCTACCTCTGGCGGAACAGCTGCTCTG GGCTGCCTCGTGAAGGACTACTTTCCTGAGCCTGTGACCGTGTC TTGGAACTCTGGCGCTCTGACATCCGGCGTGCACACATTTCCAG CTGTGCTGCAGTCCTCCGGCCTGTACTCTCTGTCCTCTGTCGTG ACCGTGCCTTCCAGCTCTCTGGGAACCCAGACCTACATCTGCA ATGTGAACCACAAGCCTTCCAACACCAAGGTGGACAAGAAGGT GGAACCCAAGTCCTGCGACAAGACCCACACCTGTCCTCCATGT CCTGCTCCAGAAGCTGCTGGCGGCCCTTCCGTGTTTCTGTTCCC TCCAAAGCCTAAGGACACCCTGATGATCTCTCGGACCCCTGAA GTGACCTGCGTGGTGGTGGATGTGTCTCACGAGGACCCAGAAG TGAAGTTCAATTGGTACGTGGACGGCGTGGAAGTGCACAACGC CAAGACCAAGCCTAGAGAGGAACAGTACAACTCCACCTACAG AGTGGTGTCCGTGCTGACCGTGCTGCACCAGGATTGGCTGAAC GGCAAAGAGTACAAGTGCAAGGTGTCCAACAAGGCCCTGCCTG CTCCTATCGAAAAGACCATCTCCAAGGCCAAGGGCCAGCCTCG AGAACCCCAGGTTTACACCCTGCCTCCAAGCCGGGAAGAGATG ACCAAGAACCAGGTGTCCCTGACCTGCCTGGTCAAGGGCTTCT ACCCTTCCGACATTGCCGTGGAATGGGAGAGCAATGGCCAGCC AGAGAACAACTACAAGACAACCCCTCCTGTGCTGGACTCCGAC GGCTCATTCTTCCTGTACTCCAAGCTGACAGTGGACAAGTCCCG GTGGCAGCAGGGCAACGTGTTCTCCTGTTCTGTGATGCACGAG GCCCTGCACAACCACTACACACAGAAGTCCCTGTCTCTGTCCCC TGGCAAAGGTGGCGGAGGATCTGGCGGAGGTGGAAGCGGCGG |
| | | AGGCGGTTCTGGTGGTGGCGGATCTCAAGTTCAGCTGGTGGAA TCTGGCGGCGGAGTGGTTCAGCCTGGAAGATCCCTGAGACTGT CTTGCGCCGCTTCTGGCTCCACCTTCAGCATCGTTGCCATGGGC TGGTACAGACAGGCTCCTGGCAAACAGCGAGAGCTGGTGGCCT CTATCATTACCGGCGACGGCGATACCAACTACGCCGACTCTGT GAAAGGCCGGTTCACCATCTCTCGGGACAACTCCAAGAACACC ATGTACCTGCAGATGAACTCCCTGAAGCCTGAGGATACAGCTG TCTACTACTGCTACGCCAGAACCGGCGAGGGATCCTCTTGGCT GGAGGGCCACGAGTACGACTACTGGGGACAGGGAACACAAGT GACAGTGTCCTCC |
| 19 | Nucleotide sequence encoding bispecific body light chain | GACATCGTGATGACCCAGTCTCCAGACTCTCTGGCCGTGTCTCT GGGCGAGAGAGCCACCATCAACTGCAAGTCCTCTCAGTCCGTG CTGTCCTCCGGCAACCAGAAGAATTACCTGGCCTGGTATCAGC AGAAGCCCGGCCAGCCTCCTAAGCTGCTGATCTACTGGGCCTC CACCAGAGAATCTGGCGTGCCCGATAGATTCTCCGGCTCTGGC TCTGGCACCGACTTTACCCTGACAATCAGCTCCCTGCAGGCCG AGGATGTGGCCGTGTACTACTGTCAGAACGCCTACTACTACCC CTTCACCTTCGGCCAGGGCACCAAGCTGGAAATCAAGAGAACC GTGGCCGCTCCTTCCGTGTTCATCTTCCCACCTTCCGACGAGCA GCTGAAGTCCGGCACAGCTTCTGTCGTGTGCCTGCTGAACAAC TTCTACCCTCGGGAAGCCAAGGTGCAGTGGAAGGTGGACAATG CCCTGCAGTCCGGCAACTCCCAAGAGTCTGTGACCGAGCAGGA CTCTAAGGACTCCACCTACAGCCTGTCCTCCACACTGACCCTGT CCAAGGCCGACTACGAGAAGCACAAGGTGTACGCCTGCGAAG TGACCCATCAGGGCCTGTCTAGCCCTGTGACCAAGTCTTTCAAC CGGGGCGAGTGT |
| 20 | Nucleotide sequence encoding Urelumab monoclonal antibody heavy chain | CAGGTCCAGCTGCAGCAGTGGGGCGCCGGACTGCTGAAGCCAA GCGAAACACTGAGCCTGACCTGCGCCGTCTACGGAGGATCCTT TTCAGGATACTATTGGAGCTGGATCAGACAGTCCCCCGAAAAG GGCCTGGAGTGGATCGGAGAGATTAACCACGGAGGATACGTG ACCTACAACCCCAGCCTGGAGAGCAGAGTGACCATCAGTGTGG ACACCTCAAAGAACCAGTTTAGCCTGAAACTGAGCAGCGTGAC CGCCGCCGACACCGCAGTCTACTACTGCGCTAGAGACTACGGC CCAGGCAACTACGACTGGTACTTCGACCTGTGGGGCAGAGGCA CCCTGGTGACAGTGAGCAGCGCCAGCACCAAGGGCCCTAGCGT GTTTCCCCTGGCCCCTTGTTCCAGAAGCACATCCGAGTCCACCG CCGCCCTGGGCTGTCTGGTGAAGGATTACTTTCCCGAGCCCGTG ACAGTGTCCTGGAACAGCGGCGCCCTGACATCCGGCGTGCACA CCTTCCCCGCCGTGCTGCAGTCCTCCGGCCTGTACAGCCTGAGC AGCGTGGTGACCGTGCCCAGCAGCAGCCTGGGCACCAAGACAT ACACCTGTAACGTGGATCACAAGCCCAGCAACACCAAGGTGGA TAAGAGGGTGGAGAGCAAGTACGGCCCCCCTTGCCCCCCCTGC CCTGCTCCTGAGTTCCTGGGCGGCCCCAGCGTGTTCCTGTTCCC CCCTAAGCCCAAGGACACACTGATGATCTCCAGAACCCCCGAG GTGACCTGTGTGGTGGTGGACGTGAGCCAGGAGGACCCTGAGG TGCAGTTTAACTGGTACGTGGACGGCGTGGAGGTGCACAATGC CAAGACAAAGCCTCGGGAGGAGCAGTTCAACTCCACATACAG AGTGGTGTCCGTGCTGACAGTGCTGCACCAGGACTGGCTGAAC GGCAAGGAGTACAAGTGTAAGGTGAGCAACAAGGGCCTGCCC AGCAGCATCGAGAAGACCATCTCCAAGGCCAAGGGCCAGCCC AGGGAGCCTCAGGTGTACACACTGCCTCCTAGCCAGGAGGAGA TGACCAAGAACCAGGTGAGCCTGACCTGCCTGGTGAAGGGCTT CTACCCTTCCGATATTGCCGTGGAGTGGGAGTCCAATGGCCAG CCCGAGAATAACTACAAGACAACCCCTCCTGTGCTGGATTCCG ACGGCTCCTTTTTCCTGTACAGCAGGCTGACAGTGGATAAGAG CAGGTGGCAGGAGGGCAACGTGTTCAGCTGTAGCGTGATGCAC GAGGCCCTGCACAACCACTACACCCAGAAGAGCCTGTCCCTGT CCCTGGGC |
| 21 | Nucleotide sequence encoding Urelumab monoclonal antibody light chain | GAGATTGTGCTGACCCAGAGCCCCGCCACACTGAGTCTGAGTC CCGGCGAGAGAGCAACACTGAGTTGTAGGGCAAGCCAGAGCG TGAGTAGCTACCTGGCCTGGTACCAGCAGAAGCCCGGCCAGGC |
| | | CCCCAGACTGCTGATTTACGACGCCTCCAACAGAGCAACCGGA ATCCCCGCCAGATTCAGCGGAAGCGGCAGCGGCACCGACTTCA CCCTGACTATCAGCAGCCTGGAACCCGAGGATTTCGCCGTCTA CTACTGCCAGCAGCGCAGCAACTGGCCCCCCGCTCTGACCTTC GGAGGAGGCACAAAGGTGGAAATCAAGCGAACTGTGGCTGCA CCATCTGTCTTCATCTTCCCGCCATCTGATGAGCAGTTGAAATC TGGAACTGCCTCTGTTGTGTGCCTGCTGAATAACTTCTATCCCA GAGAGGCCAAAGTACAGTGGAAGGTGGATAACGCCCTCCAAT CGGGTAACTCCCAGGAGAGTGTCACAGAGCAGGACAGCAAGG ACAGCACCTACAGCCTCAGCAGCACCCTGACGCTGAGCAAAGC AGACTACGAGAAACACAAAGTCTACGCCTGCGAAGTCACCCAT CAGGGCCTGAGCTCGCCCGTCACAAAGAGCTTCAACAGGGGAG AGTGC |
| 22 | Nucleotide sequence encoding 4-1BB single domain antibody heavy chain | CAGGTGCAGCTGGTTGAATCTGGTGGCGGAGTGGTGCAGCCTG GCAGATCTCTGAGACTGTCTTGTGCCGCCTCCGGCTCCACCTTT TCTATCGTGGCTATGGGCTGGTACAGACAGGCCCCTGGCAAAC AGAGAGAGCTGGTCGCCTCTATCATTACCGGCGACGGCGATAC CAACTACGCCGACTCTGTGAAGGGCAGATTCACCATCAGCCGG GACAACTCCAAGAACACCATGTACCTGCAGATGAACTCCCTGA AGCCTGAGGACACCGCCGTGTACTACTGCTACGCTAGAACCGG CGAGGGCTCCTCTTGGCTGGAAGGCCACGAGTACGATTATTGG GGCCAGGGCACCCAAGTGACCGTGTCCTCTGACAAGACCCACA CCTGTCCTCCATGTCCTGCTCCAGAAGCTGCTGGCGGCCCTTCC GTGTTTCTGTTCCCTCCAAAGCCTAAGGATACCCTGATGATCTC TCGGACCCCTGAAGTGACCTGCGTGGTGGTGGATGTGTCTCAC GAGGATCCCGAAGTGAAGTTCAATTGGTACGTGGACGGCGTGG AAGTGCACAACGCCAAGACCAAGCCTAGAGAGGAACAGTACA ACTCCACCTACAGAGTGGTGTCCGTGCTGACCGTGCTGCACCA GGATTGGCTGAACGGCAAAGAGTACAAGTGCAAGGTGTCCAA CAAGGCCCTGCCTGCTCCTATCGAAAAGACCATCTCCAAGGCC AAGGGCCAGCCTCGAGAACCCCAGGTTTACACCCTGCCTCCAA GCCGGGAAGAGATGACCAAGAACCAGGTGTCCCTGACCTGCCT CGTGAAGGGCTTCTACCCTTCCGACATTGCCGTGGAATGGGAG AGCAATGGCCAGCCAGAGAACAACTACAAGACAACCCCTCCTG TGCTGGACTCCGACGGCTCATTCTTCCTGTACTCCAAGCTGACA GTGGACAAGTCCAGATGGCAGCAGGGCAACGTGTTCTCCTGCT CCGTGATGCACGAGGCCCTGCACAATCACTACACCCAGAAGTC CCTGTCTCTGAGCCCTGGCAAG |
| 23 | Nucleotide sequence encoding Claudin 18.2 monoclonal antibody heavy chain | CAGGTGCAGCTGGTGCAGAGCGGCGCCGAGGTGAAAAAGCCC GGCGCCAGCGTGAAGGTGAGCTGCAAGGCCAGCGGCTACACCT TCACCAGCTACTACATGCACTGGGTGAGACAGGCCCCCGGCCA GGGCCTGGAGTGGATGGGCACCATCTACCCCGGCAACGGCGAC ACCAGCTACAACCAGAAGTTCCAGGGCAGAGTGACCATGACCA GAGACAAGAGCACCAGCACCGTGTACATGGAGCTGAGCAGCC TGAGAAGCGAGGACACCGCCGTGTACTTCTGCGCCAGAGGCGG CTACTACGGCAACAGCCTGGACTACTGGGGCCAGGGCACCCTG GTGACCGTGAGCAGCGCTAGCACCAAGGGCCCATCGGTCTTCC CCCTGGCACCCTCCTCCAAGAGCACCTCTGGGGGCACAGCGGC CCTGGGCTGCCTGGTCAAGGACTACTTCCCCGAACCGGTGACG GTGTCGTGGAACTCAGGCGCCCTGACCAGCGGCGTGCACACCT TCCCGGCTGTCCTACAGTCCTCAGGACTCTACTCCCTCAGCAGC GTGGTGACCGTGCCCTCCAGCAGCTTGGGCACCCAGACCTACA TCTGCAACGTGAATCACAAGCCCAGCAACACCAAGGTGGACAA GAAAGTTGAGCCCAAATCTTGTGACAAAACTCACACATGCCCA CCGTGCCCAGCACCTGAACTCCTGGGGGGACCGTCAGTCTTCC TCTTCCCCCCAAAACCCAAGGACACCCTCATGATCTCCCGGAC CCCTGAGGTCACATGCGTGGTGGTGGACGTGAGCCACGAAGAC CCTGAGGTCAAGTTCAACTGGTACGTGGACGGCGTGGAGGTGC ATAATGCCAAGACAAAGCCGCGGGAGGAGCAGTACAACAGCA CGTACCGTGTGGTCAGCGTCCTCACCGTCCTGCACCAGGACTG GCTGAATGGCAAGGAGTACAAGTGCAAGGTCTCCAACAAAGC CCTCCCAGCCCCCATCGAGAAAACCATCTCCAAAGCCAAAGGG |
| | | CAGCCCCGAGAACCACAGGTGTACACCCTGCCCCCATCCCGGG ACGAGCTGACCAAGAACCAGGTCAGCCTGACCTGCCTGGTCAA AGGCTTCTATCCCAGCGACATCGCCGTGGAGTGGGAGAGCAAT GGGCAGCCGGAGAACAACTACAAGACCACGCCTCCCGTGCTGG ACTCCGACGGCTCCTTCTTCCTCTACAGCAAGCTCACCGTGGAC AAGAGCAGGTGGCAGCAGGGGAACGTCTTCTCATGCTCCGTGA TGCATGAGGCTCTGCACAACCACTACACGCAGAAGAGCCTCTC CCTGTCTCCGGGTAAA |
| 24 | Nucleotide sequence encoding Claudin 18.2 monoclonal antibody light chain | GACATTGTGATGACCCAGAGCCCCGACTCCCTGGCCGTGAGCC TGGGAGAAAGAGCCACCATCAACTGCAAGAGCAGCCAGAGCG TGCTGAGCAGCGGCAACCAGAAAAACTACCTGGCCTGGTACCA GCAGAAGCCCGGCCAGCCCCCCAAGCTGCTGATCTACTGGGCC AGCACCAGAGAGAGCGGCGTGCCCGACAGATTCAGCGGCAGC GGCAGCGGCACCGACTTCACCCTGACCATCAGCAGCCTGCAGG CCGAGGACGTGGCCGTGTACTACTGCCAGAACGCCTACTACTA CCCCTTCACCTTCGGCCAGGGCACCAAGCTGGAGATCAAGCGT ACGGTGGCTGCACCATCTGTCTTCATCTTCCCGCCATCTGATGA GCAGTTGAAATCTGGAACTGCCTCTGTTGTGTGCCTGCTGAATA ACTTCTATCCCAGAGAGGCCAAAGTACAGTGGAAGGTGGATAA CGCCCTCCAATCGGGTAACTCCCAGGAGAGTGTCACAGAGCAG GACAGCAAGGACAGCACCTACAGCCTCAGCAGCACCCTGACGC TGAGCAAAGCAGACTACGAGAAACACAAAGTCTACGCCTGCG AAGTCACCCATCAGGGCCTGAGTTCGCCCGTCACAAAGAGCTT CAACAGGGGAGAGTGT |
| 25 | 4-1BB heavy chain variable region of single domain antibody HZ-L-Yr-16-1-AB24ME | QVQLVESGGGVVQPGRSLRLSCAASGSTFSIVAMGWYRQAPGKQ RELVASIITGDGDTNYADSVKGRFTISRDNSKNTMYLQMNSLKPE DTAVYYCYARTGYGSSELEGHEYDYWGQGTQVTVSS |
| 26 | Flexible peptide sequence-2 | GGGGSGGGGSGGGGSGGGGSG |
| 27 | Bispecific antibody Claudin 18.2x4-1BB biAb heavy chain-2 | QVQLVQSGAEVKKPGASVKVSCKASGYTFTSYYMHWVRQAPGQ GLEWMGTIYPGNGDTSYNQKFQGRVTMTRDKSTSTVYMELSSLR SEDTAVYFCARGGYYGNSLDYWGQGTLVTVSSASTKGPSVFPLA PSSKSTSGGTAALGCLVKDYFPEPVTVSWNSGALTSGVHTFPAVL QSSGLYSLSSVVTVPSSSLGTQTYICNVNHKPSNTKVDKKVEPKSC DKTHTCPPCPAPEAAGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSR EEMTKNQVSLTCLVKGFYPSDIAVEWESNGQPENNYKTTPPVLDS DGSFFLYSKLTVDKSRWQQGNVFSCSVMHEALHNHYTQKSLSLS PGKGGGGSGGGGSGGGGSGGGGSGQVQLVESGGGVVQPGRSLR LSCAASGSTFSIVAMGWYRQAPGKQRELVASIITGDGDTNYADSV KGRFTISRDNSKNTMYLQMNSLKPEDTAVYYCYARTGEGSSWLE GHEYDYWGQGTQVTVSS |
| 28 | Bispecific antibody Claudin 18.2x4-1BB biAb heavy chain-3 | QVQLVQSGAEVKKPGASVKVSCKASGYTFTSYYMHWVRQAPGQ GLEWMGTIYPGNGDTSYNQKFQGRVTMTRDKSTSTVYMELSSLR SEDTAVYFCARGGYYGNSLDYWGQGTLVTVSSASTKGPSVFPLA PSSKSTSGGTAALGCLVKDYFPEPVTVSWNSGALTSGVHTFPAVL QSSGLYSLSSVVTVPSSSLGTQTYICNVNHKPSNTKVDKKVEPKSC DKTHTCPPCPAPEAAGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSR EEMTKNQVSLTCLVKGFYPSDIAVEWESNGQPENNYKTTPPVLDS DGSFFLYSKLTVDKSRWQQGNVFSCSVMHEALHNHYTQKSLSLS PGKGGGGSGGGGSGGGGSGGGGSGQVQLVESGGGVVQPGRSLR LSCAASGSTFSIVAMGWYRQAPGKQRELVASIITGDGDTNYADSV |
| | | KGRFTISRDNSKNTMYLQMNSLKPEDTAVYYCYARTGYGSSELE GHEYDYWGQGTQVTVSS |
| 29 | Bispecific antibody Claudin 18.2x4-1BB biAb heavy chain-4 | QVQLVQSGAEVKKPGASVKVSCKASGYTFTSYYMHWVRQAPGQ GLEWMGTIYPGNGDTSYNQKFQGRVTMTRDKSTSTVYMELSSLR SEDTAVYFCARGGYYGNSLDYWGQGTLVTVSSASTKGPSVFPLA PSSKSTSGGTAALGCLVKDYFPEPVTVSWNSGALTSGVHTFPAVL QSSGLYSLSSVVTVPSSSLGTQTYICNVNHKPSNTKVDKKVEPKSC DKTHTCPPCPAPEAAGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSR EEMTKNQVSLTCLVKGFYPSDIAVEWESNGQPENNYKTTPPVLDS DGSFFLYSKLTVDKSRWQQGNVFSCSVMHEALHNHYTQKSLSLS PGKGGGGSGGGGSGGGGSGGGGSQVQLVESGGGVVQPGRSLRLS CAASGSTFSIVAMGWYRQAPGKQRELVASIITGDGDTNYADSVK GRFTISRDNSKNTMYLQMNSLKPEDTAVYYCYARTGYGSSELEG HEYDYWGQGTQVTVSS |

### Specific Models for Carrying Out the present invention

The present invention will now be described with reference to the following examples which are intended to illustrate but not to limit the present invention.

Unless otherwise indicated, the experiments and methods described in the examples were performed essentially according to conventional methods well known in the art and described in various references. For example, for conventional techniques such as immunology, biochemistry, chemistry, molecular biology, microbiology, cell biology, genomics and recombinant DNA used in the present invention, see Sambrook, Fritsch and Maniatis, MOLECULAR CLONING: A LABORATORY MANUAL, 2nd ed. (1989); CURRENT PROTOCOLS IN MOLECULAR BIOLOGY, edited by F.M. Ausubel et al., (1987); "METHODS IN ENZYMOLOGY", series, (Academic Publishing Company): "PCR 2: A PRACTICAL APPROACH", edited by M.J. MacPherson, B.D. Hames, and G.R. Taylor (1995); and ANIMAL CELL CULTURE, edited by R.I. Freshney (1987).

In addition, if the specific conditions were not specified in the examples, the conventional conditions or the conditions recommended by the manufacturer would be followed. If the manufacturer of the reagents or instruments used was not indicated, they were all conventional products that could be purchased commercially. Those skilled in the art will appreciate that the examples describe the present invention by way of example and are not intended to limit the scope of the present invention as claimed. All publications and other references mentioned herein are incorporated by reference in their entirety.

### Example 1. Cloning and expression of anti-Claudin 18.2 x 4-1BB bispecific antibody

In this example, anti-Claudin 18.2×4-1BB bispecific antibody (biAb) was constructed. Claudin 18.2x4-1BB biAb consisted of 2 polypeptide chains, and its structural diagram is depicted in Fig. 1. The heavy chain of the bispecific antibody has the amino acid sequence as set forth in SEQ ID NO: 6, which contained the amino acid sequence (SEQ ID NO: 1) of the heavy chain variable region of the monoclonal antibody anti-Claudin 18.2 mAb against claudin18 isoform 2 (Claudin 18.2), the human IgG1 Fc amino acid sequence (LALA mutation was introduced to reduce Fc function, SEQ ID NO: 4), the flexible peptide amino acid sequence (SEQ ID NO: 5), and the amino acid sequence (SEQ ID NO: 3) of the 4-1BB binding region of the single domain antibody against 4-1BB. Therein, the N-terminus of the 4-1BB heavy chain variable region amino acid sequence (SEQ ID NO: 3) of the single-domain antibody against 4-1BB is connected to the C-terminus of Fc through a flexible peptide (SEQ ID NO: 5).

The light chain of the bispecific antibody has the amino acid sequence as set forth in SEQ ID NO: 7, which contained the amino acid sequence (SEQ ID NO: 2) of the light chain variable region of the monoclonal antibody anti-Claudin 18.2 mAb against claudin18 isoform 2, and the amino acid sequence (SEQ ID NO: 8) of human κ light chain constant region (CL) at the C-terminus of the VL amino acid sequence.

ExpiCHO^{™} Expression System Kit (purchased from Thermo) was used to transfer the antibody expression plasmids, which comprised the nucleotide sequences encoding the amino acid sequences of the antibody heavy chain and light chain (the bispecific antibody Claudin 18.2x4-1BB biAb heavy chain amino acid sequence was set forth in SEQ ID NO: 6, and the bispecific antibody Claudin 18.2x4-1BB biAb light chain amino acid sequence was set forth in SEQ ID NO: 7), into Expi-CHO cells respectively. The transfection method was in accordance with the product instructions. After 5 days of culturing the cells, the supernatant was collected and the target protein was purified by sorting method using protein A magnetic beads (purchased from GenScript). The magnetic beads were resuspended in an appropriate volume of binding buffer (PBS+0.1% Tween 20, pH 7.4) (1 to 4 times the volume of magnetic beads) and added to the sample to be purified, and incubated at room temperature for 1 hour with shaking gently during the period. The sample was placed on a magnetic stand (purchased from Beaver), the supernatant was discarded, and the magnetic beads were washed three times with binding buffer. Elution buffer (0.1M sodium citrate, pH 3.2) in a volume 3 to 5 times that of the magnetic beads was added, shaken at room temperature for 5 to 10 minutes, and placed back on the magnetic stand, then the elution buffer was collected, transferred into a collection tube in which neutralization buffer (1M Tris, pH 8.54) had been added, and mixed well. The bispecific antibody of the present application was thus obtained.

ExpiCHO^{™} expression system kit (purchased from Thermo) was used to transfer the expression plasmids, containing the nucleotide sequences encoding the heavy chain and light chain of the antibody (the nucleotide sequences encoding the heavy chain and light chain of Urelumab monoclonal antibody were as set forth in SEQ ID NO: 20 and SEQ ID NO: 21, respectively; the nucleotide sequence encoding the 4-1BB single domain antibody heavy chain was set forth in SEQ ID NO: 22; and the nucleotide sequences encoding the Claudin 18.2 heavy chain and light chain were set forth in SEQ ID NO: 23 and SEQ ID NO: 24, respectively), into Expi-CHO cells. The transfection method was in accordance with the product instructions. After 5 days of culturing the cells, the supernatant was collected and the targeted protein was purified using a sorting method using protein A magnetic beads (purchased from GenScript). The magnetic beads were resuspended in an appropriate volume of binding buffer (PBS+0.1% Tween 20, pH 7.4) (1 to 4 times the volume of magnetic beads), then added to the sample to be purified, and incubated at room temperature for 1 hour with gentle shaking gently. The sample was placed on a magnetic stand (purchased from Beaver), the supernatant was discarded, and the magnetic beads were washed three times with binding buffer. Elution buffer (0.1M sodium citrate, pH 3.2) in a volume 3 to 5 times that of the magnetic beads was added, shaken at room temperature for 5 to 10 minutes, and placed back on the magnetic stand, then the elution buffer was collected, transferred into a collection tube in which neutralization buffer (1M Tris, pH 8.54) had been added, and mixed well. Urelumab monoclonal antibody, 4-1BB single domain antibody and Claudin 18.2 monoclonal antibody were thus obtained respectively.

### Example 2. Binding of bispecific antibody to Claudin 18.2

By coating human Claudin 18.2 cDNA (from GENECHEM, GCPLO162852) with lentivirus, MC38 was transfected to generate MC38 cells expressing human Claudin 18.2, named as MC38-hClaudin 18.2 cells. The expanded MC38-hClaudin 18.2 and NUGC4 (endogenously expressed hClaudin 18.2) cells were digested with 0.5 mM EDTA, washed once with culture medium, then adjusted to a cell density of 2×10⁶ cells/ml, added to a 96-well flow cytometer plate, 100 µl/well, and centrifuged for later use. The bispecific antibody was diluted with PBS, and the diluted sample in 100 µl/well was added to the 96-well flow cytometer plate with cells, incubated at 4°C for 60 minutes, and washed twice with PBS. Goat anti-human IgG-Fc (PE) (purchased from Abcam) diluted 1000 times with PBS was added (100 µl/well) and incubated at 4°C for 30 minutes, and washed twice with PBS. The cells resuspended in PBS was added, 100 µl/well, and detected on CytoFlex (Beckman) flow cytometer, and the corresponding mean fluorescence intensity (MFI) was calculated.

In the measurement experiment of the above method, the experimental results were shown in Fig. 2A. The bispecific antibody of the present invention bound to the human tumor cells endogenously expressed Claudin 18.2. Fig. 2B further showed that the bispecific antibody could bind to human Claudin 18.2 overexpressed in murine tumor cells MC38, and the binding efficacy was comparable to that of Claudin 18.2 monoclonal antibody.

### Example 3. Binding of bispecific antibody to 4-1BB

By using the PLVX vector (synthesized by Qingke) of human 4-1BB cDNA which was cloned to the multiple cloning site (MCS) by transfection, CHO cells overexpressing human 4-1BB were generated and named as CHO-h4-1BB cells. The expanded cultured CHO-h4-1BB cells was adjusted to have a cell density of 2×10⁶ cells/ml, added to a 96-well flow plate, 100 µl/well, and centrifuged for later use. The bispecific antibody was diluted with PBS, and the diluted sample in 100 µl/well was added to the 96-well flow plate with cells, incubated at 4°C for 60 min, and washed twice with PBS. Goat anti-human IgG-Fc (PE) (Abcam, ab98596) diluted 100 times with PBS was added, 100 µl/well, incubated at 4°C for 30 min, and washed twice with PBS. The cells resuspended with PBS was added, 100 µl/well, and detected on a CytoFlex (Beckman) flow cytometer, and the corresponding MFI was calculated. In the measurement experiment of the above method, the experimental results were shown in Fig. 3. The bispecific antibody of the present invention had binding activity to CHO-h4-1BB cells.

### Example 4. Activation of NF-κB signaling pathway in Jurkat cells by bispecific antibody

The target cells NUGC4 at 2×10⁴ cells/well and effector cells at 1.2×10⁵ cells/well were mixed with 4-1BB NF-κB Luciferase/Jurkat (the cells were obtained by co-transfection of Jurkat (ATCC, TIB-152^{™}) cells to express human 4-1BB vector (synthesized by Jinweizhi) and luc2P/NF-kB-RE vector (Promega, E8491)), and inoculated into a 96-well cell culture white-bottom plate, and the bispecific antibody serially diluted and anti-CD3 with a final concentration of 1 µg/mL were added and incubated for a total of 16 hours. Bio-glo luciferase assay system (Promega G7940) kit was used for color development, and a microplate reader was used to collect chemiluminescence signals. The results were shown in Fig. 4, indicating that only the bispecific antibody of the present invention activated the NF-κB signaling pathway of Jurkat cells.

### Example 5. Induction of PBMC to kill tumor cells by bispecific antibody

The target cells NUGC4 were stained using CellTrace^{™} Violet kit, inoculated into a 96-well transparent-bottom black-edged cell culture plate at 1×10⁴ cells/well, and cultured for 4 hours. The PBMCs that were thawed for one day in advance were collected, and added at 1×10⁵ cells/well into the target cell wells. The bispecific antibody was serially diluted and anti-CD3 with a final concentration of 0.5 µg/mL were mixed, and added to the cell wells for incubation. After 48 hours, Cytation 5 was used to collect DAPI fluorescence signals, and the corresponding killing intensity was calculated. The results as shown in Fig. 5, indicate that only the bispecific antibody molecule induced PBMCs to kill Claudin 18.2-positive tumor cells NUGC4 (the values on the ordinate in Fig. 5 were the results after subtracting the background value).

### Example 6. Induction of PBMC to release cytokine by bispecific antibody

The target cells NUGC4 were seeded at 1×10⁴ cells/well into a 96-well transparent-bottom black-edged cell culture plate, and cultured for 4 hours. The PBMCs that were thawed one day in advance were collected, and added at 1×10⁵ cells/well to the target cell wells. The bispecific antibody was serially diluted and anti-CD3 with a final concentration of 0.5 µg/mL were mixed, and added to the wells; after incubation for 48 hours, the supernatant was collected. The levels of IFNγ and IL-2 in the supernatant were detected using human IL-2 ELISA kit (Invitrogen, 88-7025-77) and human IFNγ (Invitrogen, 88-7316-77) ELISA kit, and the procedures recommended by the supplier were followed.

The results were shown in Fig. 6A and Fig. 6B, indicating that only the bispecific antibody molecule induced the release of IFNγ and IL-2.

### Example 7. Study on tumor inhibitory activity of bispecific antibodies

In this experiment, human MC38-hClaudin 18.2 cells and h-4-1BB transgenic C57BL/6 mouse model were used to determine the anti-tumor effects of bispecific antibodies. Sufficient MC38-hClaudin 18.2 cells (overexpressing hClaudin 18.2 on the basis of MC38 cells) were obtained by in vitro expansion, and the cells were collected after trypsin digestion, washed 3 times with PBS, counted, and inoculated at 1×10⁶ cells/mouse into female 8-week-old h-4-1BB transgenic C57BL/6 mice (purchased from Shanghai Southern Model Biotechnology Co., Ltd.) subcutaneously on the right side of the abdomen. The tumor formation under the skin of mice was observed daily, using a vernier caliper to measure the maximum width axis W and maximum long axis L of the subcutaneous tumor on the right abdomen of each animal, and an electronic balance was used to weigh each mouse. The subcutaneous tumor volume in the right abdomen of each mouse was calculated using the formula: tumor volume T=1/2×W×W×L. After excluding the mice with excessively large and small tumors, the mice were evenly divided into 4 groups according to the average tumor volume, 6 mice in each group. The mice were then grouped according to the dosing schedule in Table 2 and injected with the corresponding doses of antibodies.

The mouse tumor volume and mouse body weight were measured 2 to 3 times per week. The last measurements were taken on day 29 after inoculation of tumor cells, after which the mice were euthanized. The results were shown in Fig. 7. Compared with the PBS group, Claudin 18.2 monoclonal antibody IMAB362 had minimal inhibitory effect on tumor growth, and the combined administration of anti-Claudin 18.2 monoclonal antibody and anti-4-1BB single domain antibody also had no tumor inhibitory effect. Claudin 18.2x4-1BB biAb at the same molar dose significantly inhibited tumor growth, with all 6 mice in this group experiencing tumor regression, a marked distinction from the other 3 groups.

**Table 2. Experimental protocol for tumor inhibitory activity**

| Group | Administration type | Administration dose | Administration frequency |
|---|---|---|---|
| Group 1 | PBS | - | Once every two days, for a total of 4 doses |
| Group 2 | Claudin 18.2 mAb + 4-1BB sdAb | 3.33 mg/kg+ 1.67 mg/kg | Once every two days, for a total of 4 doses |
| Group 3 | IMAB362 | 3.33 mg/kg | Once every two days, for a total of 4 doses |
| Group 4 | Claudin 18.2×4-1BB biAb | 1 mg/kg | Once every two days, for a total of 4 doses |

### Example 8. Study on tumor inhibitory activity of bispecific antibodies (Rechallenge)

In this experiment, human MC38-hClaudin 18.2 cells and h-4-1BB transgenic C57BL/6 mouse model were used to determine the dose-dependent anti-tumor effects of bispecific antibodies. Sufficient MC38-hClaudin 18.2 cells (overexpressing hClaudin 18.2 on the basis of MC38 cells) were obtained in vitro expansion, and the cells were collected after trypsin digestion, washed 3 times with PBS, counted, and inoculated at 1×10⁶ cells/mouse into female 8-week-old h-4-1BB transgenic C57BL/6 mice (purchased from Shanghai Southern Model Biotechnology Co., Ltd.) subcutaneously on the right side of the abdomen. The tumor formation under the skin of mice was observed daily, using a vernier caliper to measure the maximum width axis W and maximum long axis L of the subcutaneous tumor on the right abdomen of each animal, and an electronic balance was used to weigh each mouse. The subcutaneous tumor volume in the right abdomen of each mouse was calculated using the formula: tumor volume T=1/2×W×W×L. After excluding the mice with excessively large and small tumors, the mice were evenly divided into 5 groups according to the average tumor volume, 6 mice in each group. The mice were grouped according to the dosing schedule in Table 3, and injected with the corresponding doses of antibodies.

The mouse tumor volume and mouse body weight were measured 2 to 3 times per week. The last measurements were taken on day 29 after inoculation of tumor cells, after which the mice were euthanized. As shown in Fig. 8, compared with the PBS group, Claudin 18.2x4-1BB biAb could significantly inhibited tumor growth in a dose-dependent manner within the dose range of 0.06 mg/kg, 0.25 mg/kg, 1 mg/kg, and 4 mg/kg, 4 mice in the 0.25 mg/kg group had tumor regression, 5 mice in the 1 mg/kg group had tumor regression, and all 6 mice in the 4 mg/kg group had tumor regression. The 15 mice with tumor regression were inoculated with enough MC38-hClaudin 18.2 tumor cells again, and the same number of MC38-hClaudin 18.2 cells were subcutaneously inoculated into 6 newly purchased human 4-1BB transgenic C57BL/6 mice as a control group. The tumors of all 6 mice in the control group grew rapidly, while the tumors of the mice with tumors regression after being given the bispecific antibody were all unable to grow, indicating that the mice in the bispecific antibody group obtained immune memory that could prevent the growth of Rechallenge tumors.

**Table 3. Experimental protocol for dose-dependent tumor inhibitory activity**

| Group | Administration type | Administration dose | Administration frequency |
|---|---|---|---|
| Group 1 | PBS | - | Once every two days, for a total of 4 doses |
| Group 2 | Claudin 18.2x4-1BB biAb | 0.06 mg/kg | Once every two days, for a total of 4 doses |
| Group 3 | Claudin 18.2x4-1BB biAb | 0.25 mg/kg | Once every two days, for a total of 4 doses |
| Group 4 | Claudin 18.2×4-1BB biAb | 1 mg/kg | Once every two days, for a total of 4 doses |
| Group 5 | Claudin 18.2×4-1BB biAb | 4 mg/kg | Once every two days, for a total of 4 doses |

Although the specific embodiments of the present invention have been described in detail, those skilled in the art will understand that various modifications and changes can be made to the details based on all teachings that have been disclosed, and these changes are within the protection scope of the present invention. The full scope of the present invention is given by the appended claims and any equivalents thereof.

## Claims

1. A bispecific antibody, which comprises a Claudin 18.2 binding entity and a 4-1 BB binding entity, wherein the Claudin 18.2 binding entity comprises two pairs of identical immunoglobulin chains, wherein, each pair of immunoglobulin chains has a light chain and a heavy chain, the heavy chain comprises a heavy chain variable region and a heavy chain constant region; the light chain comprises a light chain variable region and a light chain constant region; the 4-1 BB binding entity comprises a heavy chain variable region; and, the heavy chain variable region of the 4-1 BB binding entity is connected to the C-terminus of the heavy chain of the Claudin 18.2 binding entity; wherein,
the Claudin 18.2 binding entity comprises: a VH CDR1 or variant thereof, a VH CDR2 or variant thereof, and a VH CDR3 or variant thereof contained in the heavy chain variable region (VH) as set forth in SEQ ID NO: 1; and/or, a VL CDR1 or variant thereof, a VL CDR2 or variant thereof, and a VL CDR3 or variant thereof contained in the light chain variable region (VL) as set forth in SEQ ID NO: 2;
the 4-1 BB binding entity comprises: a VH CDR1 or variant thereof, a VH CDR2 or variant thereof, and a VH CDR3 or variant thereof contained in the heavy chain variable region (VH) as set forth in SEQ ID NO: 3 or 25;
wherein, the variant has a substitution, deletion or addition of one or several amino acids (e.g., a substitution, deletion or addition, such as a conservative substitution, of 1, 2 or 3 amino acids) as compared to the sequence from which it is derived; preferably, the substitution is a conservative substitution;
preferably, the three CDRs contained in the VH and/or the three CDRs contained in the VL are defined by the Kabat, IMGT or Chothia numbering system.

2. The antibody or antigen-binding fragment thereof according to claim 1, which has one or two characteristics selected from the following:
(1) the Claudin 18.2 binding entity comprises:
a heavy chain variable region (VH) comprising the following 3 complementarity determining regions (CDRs): a VH CDR1 with the sequence as set forth in SEQ ID NO: 9, a VH CDR2 with the sequence as set forth in SEQ ID NO: 10, and a VH CDR3 with the sequence as set forth in SEQ ID NO: 11; and/or a light chain variable region (VL) comprising the following 3 complementarity determining regions (CDRs): a VL CDR1 with the sequence as set forth in SEQ ID NO: 12, a VL CDR2 with the sequence as set forth in SEQ ID NO: 13, and a VL CDR3 with the sequence as set forth in SEQ ID NO: 14; wherein the CDRs are defined by the IMGT numbering system; or
(2) the 4-1 BB binding entity comprises:
a heavy chain variable region (VH) comprising the following 3 complementarity determining regions (CDRs): a VH CDR1 with the sequence as set forth in SEQ ID NO: 15, a VH CDR2 with the sequence as set forth in SEQ ID NO: 16, and a VH CDR3 with the sequence as set forth in SEQ ID NO: 17; wherein the CDRs are defined by the IMGT numbering system.

3. A bispecific antibody, which comprises a Claudin 18.2 binding entity and a 4-1 BB binding entity, wherein the Claudin 18.2 binding entity comprises two pairs of identical immunoglobulin chains, wherein, each pair of immunoglobulin chains has a light chain and a heavy chain, the heavy chain comprises a heavy chain variable region and a heavy chain constant region; the light chain comprises a light chain variable region and a light chain constant region; the 4-1 BB binding entity comprises a heavy chain variable region; and, the heavy chain variable region of the 4-1 BB binding entity is connected to the C terminus of the heavy chain of the Claudin 18.2 binding entity;
wherein, the heavy chain variable region (VH) of the Claudin 18.2 binding entity comprises the following 3 complementarity determining regions (CDRs):
(i) a VH CDR1, which consists of the following sequence: SEQ ID NO: 9, or a sequence having a substitution, deletion or addition of one or several amino acids (e.g., a substitution, deletion or addition of 1, 2 or 3 amino acids) as compared thereto,
(ii) a VH CDR2, which consists of the following sequence: SEQ ID NO: 10, or a sequence having a substitution, deletion or addition of one or several amino acids (e.g., a substitution, deletion or addition of 1, 2 or 3 amino acids) as compared thereto, and
(iii) a VH CDR3, which consists of the following sequence: SEQ ID NO: 11, or a sequence having a substitution, deletion or addition of one or several amino acids (e.g., a substitution, deletion or addition of 1, 2 or 3 amino acids) as compared thereto,
the light chain variable region (VL) of the Claudin 18.2 binding entity comprises the following 3 complementarity determining regions (CDRs):
(iv) a VL CDR1, which consists of the following sequence: SEQ ID NO: 12, or a sequence having a substitution, deletion or addition of one or several amino acids (e.g., a substitution, deletion or addition of 1, 2 or 3 amino acids) as compared thereto,
(v) a VL CDR2, which consists of the following sequence: SEQ ID NO: 13, or a sequence having a substitution, deletion or addition of one or several amino acids (e.g., a substitution, deletion or addition of 1, 2 or 3 amino acids) as compared thereto, and
(vi) a VL CDR3, which consists of the following sequence: SEQ ID NO: 14, or a sequence having a substitution, deletion or addition of one or several amino acids (e.g., a substitution, deletion or addition of 1, 2 or 3 amino acids) as compared thereto;
the heavy chain variable region of the 4-1 BB binding entity comprises the following 3 complementarity determining regions (CDRs):
(vii) a VH CDR1, which consists of the following sequence: SEQ ID NO: 15, or a sequence having a substitution, deletion or addition of one or several amino acids (e.g., a substitution, deletion or addition of 1, 2 or 3 amino acids) as compared thereto,
(viii) a VH CDR2, which consists of the following sequence: SEQ ID NO: 16, or a sequence having a substitution, deletion or addition of one or several amino acids (e.g., a substitution, deletion or addition of 1, 2 or 3 amino acids) as compared thereto, and
(ix) a VH CDR3, which consists of the following sequence: SEQ ID NO: 17, or a sequence having a substitution, deletion or addition of one or several amino acids (e.g., a substitution, deletion or addition of 1, 2 or 3 amino acids) as compared thereto;
preferably, the Claudin 18.2 binding entity comprises: the following 3 heavy chain CDRs: a VH CDR1 with the sequence as set forth in SEQ ID NO: 9, a VH CDR2 with the sequence as set forth in SEQ ID NO: 10, a VH CDR3 with the sequence as set forth in SEQ ID NO: 11; and/or, the following 3 light chain CDRs: a VL CDR1 with the sequence as set forth in SEQ ID NO: 12, a VL CDR2 with the sequence as set forth in SEQ ID NO: 13, and a VL CDR3 with the sequence as set forth in SEQ ID NO: 14;
preferably, the heavy chain variable region (VH) of the 4-1 BB binding entity comprises: the following 3 heavy chain CDRs: a VH CDR1 with the sequence as set forth in SEQ ID NO: 15, a VH CDR2 with the sequence as set forth in SEQ ID NO: 16, and a VH CDR3 with the sequence as set forth in SEQ ID NO: 17;
preferably, the heavy chain variable region further comprises a framework region sequence derived from human (e.g., a human immunoglobulin).

4. The bispecific antibody according to claim 3, wherein the Claudin 18.2 binding entity comprises:
(a) a heavy chain variable region (VH) comprising an amino acid sequence selected from the following:
(i) a sequence as set forth in SEQ ID NO: 1;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 1; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 1;
and/or,
(b) a light chain variable region (VL) comprising an amino acid sequence selected from the following:
(iv) a sequence as set forth in SEQ ID NO: 2;
(v) a sequence having a substitution, deletion or addition of one or several amino acids (e.g., a substitution, deletion or addition of 1, 2, 3, 4 or 5 amino acids) as compared to the sequence as set forth in SEQ ID NO: 2; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 2;
preferably, the substitution described in (ii) or (v) is a conservative substitution;
preferably, the Claudin 18.2 binding entity comprises: a VH having the sequence as set forth in SEQ ID NO: 1 and a VL having the sequence as set forth in SEQ ID NO: 2.

5. The bispecific antibody according to claim 3 or 4, wherein the 4-1 BB binding entity comprises:
(a) a heavy chain variable region (VH) comprising an amino acid sequence selected from the following:
(i) a sequence as set forth in SEQ ID NO: 3 or 25;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 3 or 25; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 3 or 25.
preferably, the substitution described in (ii) is a conservative substitution.

6. The bispecific antibody according to any one of claims 1 to 5, wherein the Claudin 18.2 binding entity further comprises:
(a) a human immunoglobulin heavy chain constant region (CH) or variant thereof, wherein the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived; and
(b) a human immunoglobulin light chain constant region (CL) or variant thereof, wherein the variant has a conservative substitution of up to 20 amino acids (e.g., a conservative substitution of up to 15, up to 10, or up to 5 amino acids; for example, a conservative substitution of 1, 2, 3, 4 or 5 amino acids) as compared to the wild-type sequence from which it is derived;
preferably, the heavy chain constant region is an IgG heavy chain constant region, such as an IgGl, IgG2, IgG3 or IgG4 heavy chain constant region; preferably, the light chain constant region is a κ or λ light chain constant region;
preferably, the Claudin 18.2 binding entity comprises a light chain constant region (CL) as set forth in SEQ ID NO: 8;
preferably, the bispecific antibody further comprises an Fc fragment that does not bind to an Fc receptor (FcR); alternatively, comprises an Fc fragment that has reduced effector function, wherein the effector function is antibody-dependent cell-mediated cytotoxicity (ADCC), complement-dependent cytotoxicity (CDC), or antibody-dependent cellular phagocytosis (ADCP);
preferably, the Fc fragment is selected from any one of the following mutants:
(a) an IgG1 Fc fragment having L235E mutation;
(b) an IgG1 Fc fragment having L234A and/or L235A mutations;
(c) an IgG1 Fc fragment having P329G or P329A mutation;
(d) an IgG4 Fc fragment having F234A and/or L235A or L235E mutations;
(e) an IgG2 Fc fragment having H268Q, V309L, A330S and/or P331S mutations; or
(f) an IgG2 Fc fragments having V234A, G237A, P238S, H268A, V309L, A330S and/or P331S mutations;
preferably, the Fc fragment is an IgGl Fc fragment having L234A and L235A mutations;
preferably, the Fc fragment has the sequence as set forth in SEQ ID NO: 4.

7. The bispecific antibody according to any one of claims 1 to 6, wherein the bispecific antibody further comprises a peptide linker, and the Claudin 18.2 binding entity and the 4-1 BB binding entity are connected through a peptide linker;
preferably, the C-terminus of the heavy chain constant region of the Claudin 18.2 binding entity and the N-terminus of the heavy chain variable region of the 4-1 BB binding entity are connected through a peptide linker;
preferably, the peptide linker has the sequence as set forth in SEQ ID NO: 5 or 26;
preferably, the heavy chain of the bispecific antibody has the amino acid sequence as set forth in SEQ ID NO: 6, 27, 28 or 29;
preferably, the light chain of the bispecific antibody has the amino acid sequence as set forth in SEQ ID NO: 7.

8. An isolated nucleic acid molecule, which encodes the bispecific antibody or heavy chain and/or light chain thereof according to any one of claims 1 to 7, or the heavy chain variable region and/or light chain variable region of a Claudin 18.2 binding entity, or the heavy chain variable region of a 4-1 BB binding entity.

9. A vector, which comprises the isolated nucleic acid molecule according to claim 8; preferably, the vector is a cloning vector or an expression vector.

10. A host cell, which comprises the isolated nucleic acid molecule according to claim 8 or the vector according to claim 9.

11. An immunoconjugate, which comprises the bispecific antibody according to any one of claims 1 to 7 and a therapeutic agent connected to the bispecific antibody;
preferably, the therapeutic agent is selected from cytotoxic agents;
preferably, the therapeutic agent is selected from the group consisting of alkylating agent, mitotic inhibitor, anti-tumor antibiotic, antimetabolite, topoisomerase inhibitor, tyrosine kinase inhibitor, radionuclide agent, and any combination thereof;
preferably, the immunoconjugate is an antibody-drug conjugate (ADC).

12. A pharmaceutical composition, which comprises the bispecific antibody according to any one of claims 1 to 7 or the immunoconjugate according to claim 11, and a pharmaceutically acceptable carrier and/or excipient;
preferably, the pharmaceutical composition further comprises an additional pharmaceutically active agent;
preferably, the additional pharmaceutically active agent is a drug with anti-tumor activity, such as an alkylating agent, a mitotic inhibitor, an anti-tumor antibiotic, an antimetabolite, a topoisomerase inhibitor, a tyrosine kinase inhibitor, a radioactive nuclide, a radiosensitizer, an anti-angiogenic agent, a cytokine, a molecular targeted drug, an immune checkpoint inhibitor or an oncolytic virus;
preferably, the bispecific antibody or immunoconjugate and the additional pharmaceutically active agent are provided as separate components or as components of the same composition.

13. A method for inhibiting the growth of a tumor cell expressing Claudin 18.2 and/or killing the tumor cell, which comprises contacting the tumor cell with an effective amount of the bispecific antibody according to any one of claims 1 to 7, or the immunoconjugate according to claim 11, or the pharmaceutical composition according to claim 12.

14. Use of the bispecific antibody according to any one of claims 1 to 7, or the immunoconjugate according to claim 11 in the manufacture of a medicament for preventing and/or treating a tumor in a subject (e.g., a human);
preferably, the medicament further comprises an additional pharmaceutically active agent;
preferably, the additional pharmaceutically active agent is a drug with anti-tumor activity, such as an alkylating agent, a mitotic inhibitor, an anti-tumor antibiotic, an antimetabolite, a topoisomerase inhibitor, a tyrosine kinase inhibitor, a radioactive nuclide, a radiosensitizer, an anti-angiogenic agent, a cytokine, a molecular targeted drug, an immune checkpoint inhibitor or an oncolytic virus;
preferably, the tumor expresses Claudin 18.2;
preferably, the tumor involves a tumor cell expressing Claudin 18.2; preferably, the Claudin 18.2 is expressed on the surface of the tumor cell;
preferably, the tumor is selected from the group consisting of gastric cancer, liver cancer, biliary tract cancer, renal cell cancer, pancreatic cancer, non-small cell lung cancer, mesothelioma, ovarian cancer, testicular cancer, endometrial cancer, lung cancer, esophageal cancer, pancreatic cancer, bronchial cancer, breast cancer, ear-nose-throat (ENT) cancer, colon cancer, head and neck cancer, gallbladder cancer;
preferably, the subject is a mammal, such as a human.

15. Use of the bispecific antibody according to any one of claims 1 to 7, or the immunoconjugate according to claim 11, or the pharmaceutical composition according to claim 12 in the manufacture of a kit, wherein the kit is used for:
(1) recognizing or binding to a cell expressing Claudin 18.2;
(2) recognizing or binding to a cell expressing 4-1BB;
(3) activating a NF-κB signaling pathway of a cell;
(4) inducing a cell (e.g., an immune cell) to activate tumor-killing activity;
(5) inducing a cell (e.g., an immune cells) to secrete a cytokine (e.g., IFNγ and IL-2);
(6) any combination of (1) to (5).
